# EUROPÄISCHE PATENTSCHRIFT

(11) **EP 3 102 168 B1**
(45) Veröffentlichungstag und Bekanntmachung des Hinweises auf die Patenterteilung: **18.09.2019**
(21) Anmeldenummer: 15704485.0
(22) Anmeldetag: 30.01.2015
(51) Int. Cl.: A61F 9/02

(54) **BRILLE**
GOGGLES
LUNETTES

(30) Priorität: 04.02.2014 DE 102014201968
(43) Veröffentlichungstag der Anmeldung: 14.12.2016
(73) Patentinhaber: Uvex Arbeitsschutz GmbH, 90766 Fürth (DE)
(72) Erfinder: FRANK, Harald, 90411 Nürnberg (DE); BRANDIS, Michael, 90429 Nürnberg (DE); KAILAS, Johannes, 90765 Fürth (DE); TÄUBER, Manfred, 90579 Langenzenn (DE)
(74) Vertreter: Rau, Schneck & Hübner Patentanwälte Rechtsanwälte PartGmbB
(86) Internationale Anmeldenummer: PCT/EP2015/051992
(87) Internationale Veröffentlichungsnummer: WO 2015/117903

(56) Entgegenhaltungen:
- US-A1- 2003 019 018
- US-A1- 2007 028 371
- US-A1- 2009 222 979
- US-B1- 8 601 617

## Beschreibung

Die Erfindung betrifft eine Brille, insbesondere eine Arbeitsschutzbrille, gemäß Anspruchs 1. Alternativ ist die Brille beispielsweise als Sportbrille ausgeführt.

Aus dem Stand der Technik sind bereits mehrteilige Brillen bekannt. Nachteilig an diesen bekannten Brillen ist oftmals, dass deren Montage zeitaufwendig und kompliziert ist. Im Allgemeinen ist es erforderlich, die Verbindungsstellen zwischen den einzelnen Komponenten der Brille äußerst stabil und massiv auszubilden.

Eine in der US 2003/0019018 A1 offenbarte Brille hat einen Rahmen, zwei Scheibenanordnungen und ein Kopfband. Der Rahmen umfasst seitliche Clipse mit äußeren Aussparungen und inneren Stegen. Die erste Scheibenanordnung hat Seitenlaschen mit äußeren Stegen und inneren Nuten. Sie wird an dem Rahmen festgelegt, indem deren Seitenlaschen in die Clipse eingeführt werden, bis mindestens eine der Nuten mit dem jeweiligen inneren Steg in Eingriff steht. Durch die Wahl der Nut ist die erste Scheibenanordnung in unterschiedlichen Positionen zu dem Rahmen anordenbar. Die zweite Scheibeanordnung umfasst Seitenlaschen mit einem Steg und einer inneren Nut. Die Seitenlaschen der zweiten Scheibenanordnung werden in die Clipse über die Seitenlaschen der ersten Scheibenanordnung eingeführt. Die innere Nut der zweiten Scheibenanordnung steht in Eingriff mit einem der Stege der ersten Scheibenanordnung. Die erste und zweite Scheibenanordnung werden an dem Rahmen durch Clipse des Kopfbandes festgelegt. Die Clipse des Kopfbandes werden in die Clipse des Rahmens über die Seitenlaschen der ersten Scheibenanordnung, wenn nur eine Scheibe verwendet wird, oder über die Seitenlaschen der zweiten Scheibenanordnung eingeführt, falls beide Scheiben eingesetzt werden.

Eine aus der US 2009/0222979 A1 bekannte Brillenanordnung umfasst eine Scheibe, einen starren Rahmen, einen nachgiebigen Rahmen und ein Kopfband mit endseitig angeordneten Schnallen. Die Scheibe hat seitlich vorspringende Laschen mit einem Vorsprung. Seitlich hat der starre Rahmen jeweils eine äußere Aussparung. Der nachgiebige Rahmen weist seitlich jeweils eine Durchgangsöffnung auf. Jede Schnalle hat ein Eingriffselement und eine Öffnung. Die Eingriffselemente werden in die Durchgangsöffnungen eingeführt, während die Öffnungen von den Vorsprüngen durchsetzt werden.

Die US 2007/0028371 A1 offenbart eine Schwimmbrille mit einem Rahmen, einem Kissen, Scheiben, einem Verbindungsabschnitt, einem Befestigungsabschnitt und einem Kopfband. Ein seitliches Deckelement umgibt Befestigungselemente, die an zwei Rahmenteilen angeordnet sind. Ein Einstellteil ist mit dem jeweiligen Befestigungselement verbindbar. Das Kopfband ist an den Einstellteilen befestigt.

Aus der US 8,601,617 B1, gegenüber welcher der Anspruch 1 abgegrenzt ist, sind beispielsweise verschiedene Masken bekannt, an welchen unterschiedliche Einrichtungen, wie ein Datenübertragungsgerät, ein Einglas, eine Linse oder eine Hörmuschel, befestigbar sind.

Die US 7,882,575 B2 offenbart eine Schutzbrille mit einer Scheibe, einem Rahmen und einem Kopfband.

Der Erfindung liegt daher die Aufgabe zugrunde, eine mehrteilige Brille bereitzustellen, die einfach montierbar und/oder demontierbar ist. Ferner soll die Brille insbesondere auch mechanisch stark beanspruchbar sein, was insbesondere auch für die Verbindungsstellen zwischen den einzelnen Komponenten gilt. Die Brille soll außerdem vorzugsweise filigran wirken und optisch ansprechend sein.

Diese Aufgabe wird erfindungsgemäß durch eine Brille mit den in dem unabhängigen Anspruch 1 angegebenen Merkmalen gelöst. Der Kern der Erfindung liegt darin, dass die Brille verschiedene Kopplungs-Einrichtungen zum koppelnden, lösbaren Verbinden der Sichtscheiben-Anordnung, des Rahmens und der Kopf-Halteanordnung umfasst. An der Sichtscheiben-Anordnung und dem Rahmen sowie der Kopf-Halteanordnung sind jeweils Kopplungs-Einrichtungen angeordnet, die Kopplungs-Anordnungen zum Koppeln derselben bilden. Im zusammengesetzten Zustand der Brille ist die zweite Kopplungs-Einrichtung und dritte Kopplungs-Einrichtung der jeweiligen Kopplungs-Anordnung über das zugehörige Halte-Mittel und Halte-Gegenmittel koppelnd miteinander verbunden. Die erste Kopplungs-Einrichtung steht mit der jeweiligen zweiten Kopplungs-Einrichtung und dritten Kopplungs-Einrichtung in koppelnder Verbindung.

Der zur Demontage zu betätigende überstehende Bereich des Halte-Körpers ist nur von der Innenseite der Brille aus zugänglich. Dies dient insbesondere der Sicherheit, da so eine unbeabsichtigte Demontage der Brille, zum Beispiel einen von außen auf die Brille auftreffenden Fremdkörper, vermieden wird.

Der mindestens eine Gelenk-Vorsprung ist vorzugsweise als länglicher Gelenk-Steg ausgeführt. Alternativ bzw. zusätzlich ist dieser beispielsweise an der jeweiligen ersten Kopplungs-Einrichtung angeordnet.

Es ist von Vorteil, wenn die Sichtscheiben-Anordnung, der Rahmen und die Kopf-Halteanordnung vor deren Zusammensetzung separate bzw. getrennte Teile bzw. Komponenten der Brille sind. Diese sind so einfach austauschbar.

Es ist zweckmäßig, wenn die Sichtscheiben-Anordnung eine einzige, durchgehende einstückige Sichtscheibe hat. Alternativ sind zwei einzelne Sichtscheiben vorhanden, die beispielsweise über eine Brücke, einen Nasensteg oder dergleichen miteinander verbunden sind. Die Sichtscheiben-Anordnung ist vorzugsweise im Wesentlichen formstabil ausgeführt.

Günstigerweise ist der Rahmen umfangsseitig geschlossen. Vorzugsweise erstreckt sich der Rahmen zumindest entlang eines Teils, bevorzugter mindestens entlang eines Großteils eines Umfangs, der Sichtscheiben-Anordnung. Der Rahmen trägt vorzugsweise mindestens ein Dichtelement zur dichten Anlage an dem Gesicht eines Trägers. Das mindestens eine Dichtelement ist vorzugsweise weich bzw. nachgiebig ausgeführt.

Die Kopf-Halteanordnung ist günstigerweise als Kopf-Halteband ausgeführt, das im Einsatz um den Hinterkopf eines Trägers läuft. Das Kopf-Halteband ist vorzugsweise elastisch. Alternativ ist die Kopf-Halteanordnung beispielsweise als Bügel-Anordnung ausgeführt, die zwei Brillenbügel umfasst. Es ist von Vorteil, wenn die Brillenbügel dann in bekannter Weise einklappbar und ausklappbar sind.

Es ist von Vorteil, wenn die ersten Kopplungs-Einrichtungen jeweils einstückig ausgeführt sind und aus Kunststoff gebildet sind.

Günstigerweise sind die zweiten Kopplungs-Einrichtungen jeweils einstückig ausgeführt und aus Kunststoff gebildet. Wenn die Kopf-Halteanordnung als Kopf-Halteband ausgeführt ist, ist das Kopf-Halteband vorzugsweise durch jede zweite Kopplungs-Einrichtung hindurchgeführt und zum örtlichen Festlegen der jeweiligen zweiten Kopplungs-Einrichtung dort schlaufenartig ausgeführt. Andere Befestigungen zwischen dem Kopf-Halteband und den zweiten Kopplungs-Einrichtungen sind alternativ möglich.

Vorzugsweise sind die dritten Kopplungs-Einrichtungen jeweils einstückig und aus Kunststoff gebildet. Die dritten Kopplungs-Einrichtungen sind günstigerweise jeweils einstückig mit dem Rahmen ausgebildet.

Es ist von Vorteil, wenn die Brille bezüglich einer Mittel- bzw. Symmetrieebene symmetrisch ausgeführt ist.

Vorzugsweise verschließen die Halte-Körper die jeweiligen Teil-Aufnahmen in Richtung auf die Mittelebene der Brille. Der Halte-Körper kann ein oder mehrere Halte-Mittel aufweisen.

Weitere vorteilhafte Ausgestaltungen der Erfindung sind in den Unteransprüchen angegeben.

Die Ausgestaltung gemäß dem Unteranspruch 2 führt zu einer äußerst belastbaren und sicheren örtlichen Fixierung der ersten Kopplungs-Einrichtung gegenüber der zweiten Kopplungs-Einrichtung und dritten Kopplungs-Einrichtung der jeweiligen Kopplungs-Anordnung. Die zweite Kopplungs-Einrichtung und die dritte Kopplungs-Einrichtung der jeweiligen Kopplungs-Anordnung sind im zusammengesetzten Zustand der Brille im Wesentlichen unbeweglich zueinander.

Der Halte-Vorsprung gemäß dem Unteranspruch 3 bildet vorzugsweise einen Riegel. Er ist günstigerweise stegartig ausgeführt. Vorzugsweise befindet sich der Halte-Vorsprung im zusammengesetzten Zustand der Brille innerhalb der jeweiligen Teil-Aufnahme. Bevorzugt steht der Halte-Vorsprung im zusammengesetzten Zustand der Brille nicht gegenüber der benachbarten dritten Kopplungs-Einrichtung nach seitlich außen über. Insbesondere steht der Halte-Vorsprung im zusammengesetzten Zustand der Brille nicht über einer gedachten äußeren seitlichen Begrenzungsfläche der dritten Kopplungs-Einrichtung vor. Vorzugsweise endet der Halte-Vorsprung im zusammengesetzten Zustand der Brille sogar ein Stück unterhalb der gedachten äußeren seitlichen Begrenzungsfläche der dritten Kopplungs-Einrichtung.

Gemäß dem Unteranspruch 4 springen der Halte-Vorsprung und das Halte-Mittel der jeweiligen Kopplungs-Anordnung im zusammengesetzten Zustand der Brille in gegensinnigen Richtungen bezüglich einer Mittelebene der jeweiligen Kopplungs-Anordnung vor. Es ist von Vorteil, wenn der Halte-Vorsprung und das Halte-Mittel der jeweiligen Kopplungs-Anordnung in Blickrichtung der Brille versetzt zueinander angeordnet sind.

Die Ausgestaltung gemäß dem Unteranspruch 5 verhindert insbesondere ein Verkippen der zweiten Kopplungs-Einrichtung bzw. dritten Kopplungs-Einrichtung gegenüber der ersten Kopplungs-Einrichtung der jeweiligen Kopplungs-Anordnung. Die (recht-)eckige Ausgestaltung der ersten Kopplungs-Einrichtung ist insbesondere im Wesentlichen in einer zur Mittelebene der Brille parallelen Ebene gegeben. In dieser Ebene hat die erste Kopplungs-Einrichtung eine insbesondere (recht-)eckige Querschnittsfläche oder Kontur, wobei die Ecken vorzugsweise abgerundet sein können. Anstelle einer rechteckigen Ausgestaltung kann die erste Kopplungs-Einrichtung aber z.B. trapezförmig, dreieckig, fünfeckig, sechseckig oder dergleichen sein und eine entsprechende Querschnittsfläche oder Kontur haben. Alternativ kann die erste Kopplungs-Einrichtung auch insbesondere elliptisch, oval, eiförmig oder dergleichen sein und eine entsprechende Querschnittsfläche oder Kontur haben. Die erste Kopplungs-Einrichtung wäre demnach also vorzugsweise unrund ausgeführt. Ebenso kann die erste Kopplungs-Einrichtung aber z.B. auch rund sein und eine entsprechende Querschnittsfläche oder Kontur haben. Kombinationen der genannten unterschiedlichen Ausgestaltungen sind ebenfalls möglich.

Es ist von Vorteil, wenn die Rahmen-Teil-Aufnahmen und die Kopf-Halteanordnungs-Teil-Aufnahmen jeweils an die Form bzw. Kontur der jeweiligen ersten Kopplungs-Einrichtung angepasst sind, um vorzugsweise jeweils eine formschlüssige Verbindung zwischen den ersten Kopplungs-Einrichtungen und den jeweiligen Gesamt-Aufnahmen zu ermöglichen. Wenn also beispielsweise die ersten Kopplungs-Einrichtungen jeweils rund sind, so sind die Kopf-Halteanordnungs-Teil-Aufnahmen und Rahmen-Teil-Aufnahmen jeweils bevorzugt kreissegmentförmig, bevorzugter halbkreisförmig, ausgebildet.

Die in dem Unteranspruch 6 angegebene Durchgangs-Öffnung bildet quasi auch eine Montage- bzw. Demontage-Öffnung.

Die Ausgestaltung des Halte-Körpers gemäß dem Unteranspruch 6 erlaubt eine besonders leichtgängige Verschwenkung des jeweiligen Halte-Körpers. Es ist von Vorteil, wenn der gegenüber der Durchgangs-Öffnung überstehende Bereich des Halte-Körpers gut von Hand erreichbar ist.

Die Ausgestaltung gemäß dem Unteranspruch 7 führt zu einer Kopplungs-Anordnung, die äußerst betriebssicher bzw. funktionssicher ist. Der Halte-Körper ist insbesondere derart ausgestaltet, dass er im zusammengesetzten Zustand der Brille automatisch in seine Halte-Stellung federt. Zum Überführen des jeweiligen Halte-Körpers von dessen Halte-Stellung in dessen Freigabe-Stellung ist insbesondere eine entsprechende Verstellkraft entgegen der Federkraft auf den Halte-Körper aufzubringen.

Die gemäß dem Unteranspruch 9 innere Anordnung des jeweiligen Halte-Mittels in der jeweiligen dritten Kopplungs-Einrichtung führt zu einer äußerst geschützten Anordnung des entsprechenden Halte-Mittels. Es ist insbesondere so vor mechanischen Beanspruchungen geschützt. Es ist von Vorteil, wenn auch das zugehörige Halte-Gegenmittel geschützt innenseitig an der jeweiligen dritten Kopplungs-Einrichtung angeordnet ist.

Insbesondere ist die Halte-Fläche gemäß dem Unteranspruch 10 länglich ausgeführt. Diese liegt günstigerweise in einer Ebene, die senkrecht oder schräg zu einer Symmetrieebene der Brille verläuft.

Die Halte-Gegenfläche gemäß dem Unteranspruch 11 verläuft im zusammengesetzten Zustand der Brille benachbart zu der zugehörigen Halte-Fläche und liegt dann vorzugsweise flächig an dieser an.

Die Ausgestaltung gemäß dem Unteranspruch 12 erlaubt ein Verschwenken des jeweiligen Halte-Körpers in Richtung auf die benachbart und in diesem Bereich insbesondere beabstandet angeordnete erste Kopplungs-Einrichtung, um die Freigabe-Stellung des Halte-Körpers zu erreichen.

Die Ausgestaltung gemäß dem Unteranspruch 13 führt zu einer äußerst funktionssicheren und betriebssicheren Kopplungs-Anordnung. Die Sichtscheiben-Anordnung und die dritten Kopplungs-Einrichtungen sind so zueinander fixiert.

Die Ausgestaltung gemäß dem Unteranspruch 14 erlaubt eine äußerst sichere bzw. verliersichere Verbindung zwischen den ersten Kopplungs-Einrichtungen und der Sichtscheiben-Anordnung. Eine derartige Brille ist äußerst montagefreundlich und kostengünstig herstellbar.

Nachfolgend werden unter Bezugnahme auf die beigefügte Zeichnung zwei bevorzugte Ausführungsformen der Erfindung beispielhaft beschrieben. Dabei zeigen:
- Fig. 1: eine Explosionsansicht einer erfindungsgemäßen Brille gemäß einer ersten Ausführungsform,
- Fig. 2: eine Ansicht, die die in Fig. 1 dargestellte Brille im zusammengesetzten bzw. montierten Zustand zeigt,
- Fig. 3: einen Schnitt durch die in Fig. 2 veranschaulichte, zusammengesetzte Brille,
- Fig. 4: eine aufgeschnittene Ansicht der in den Fig. 2 und 3 gezeigten, zusammengesetzten Brille,
- Fig. 5: eine perspektivische Ansicht einer erfindungsgemäßen Brille im zusammengesetzten Zustand gemäß einer zweiten Ausführungsform, und
- Fig. 6: eine Schnittansicht der in Fig. 5 gezeigten, zusammengesetzten Brille.

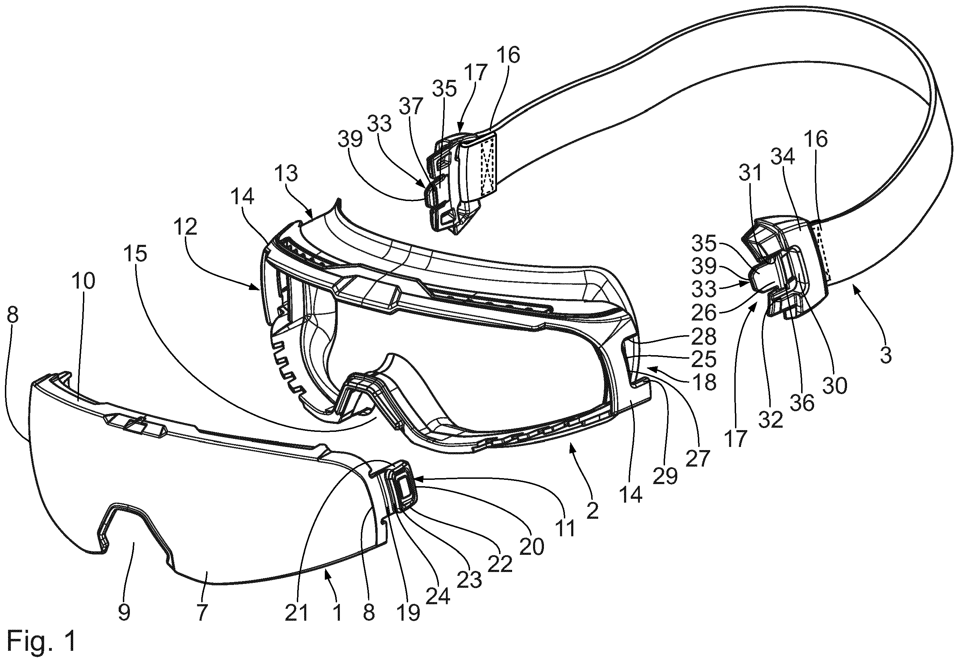

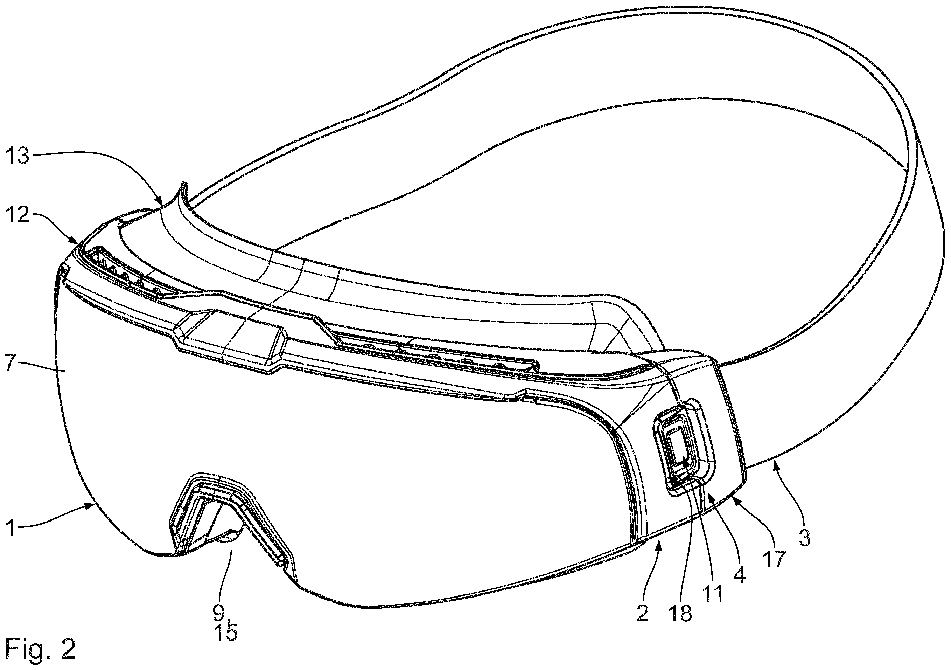

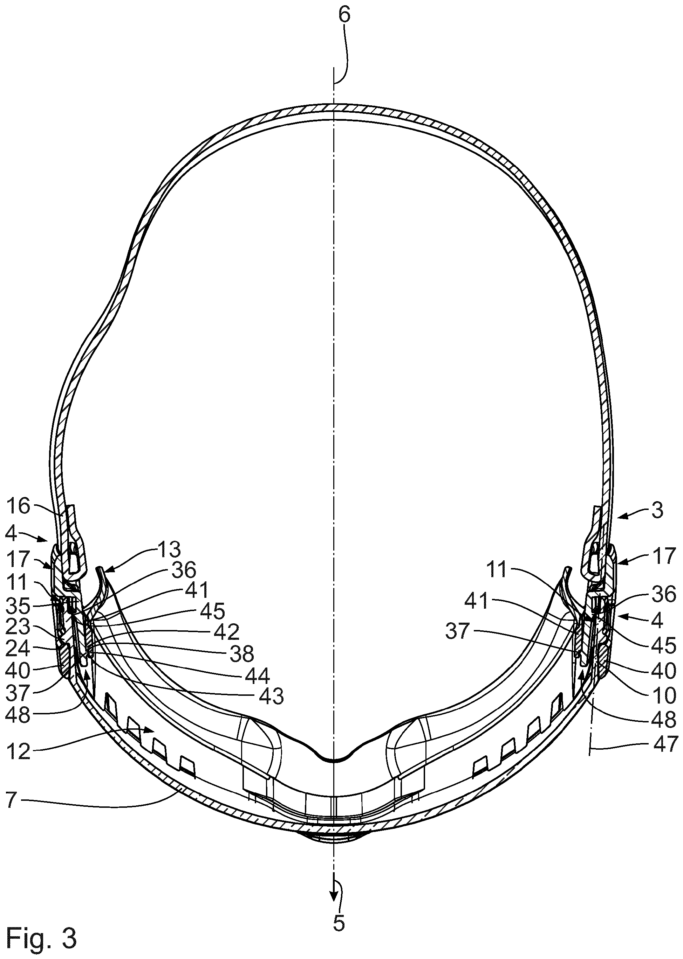

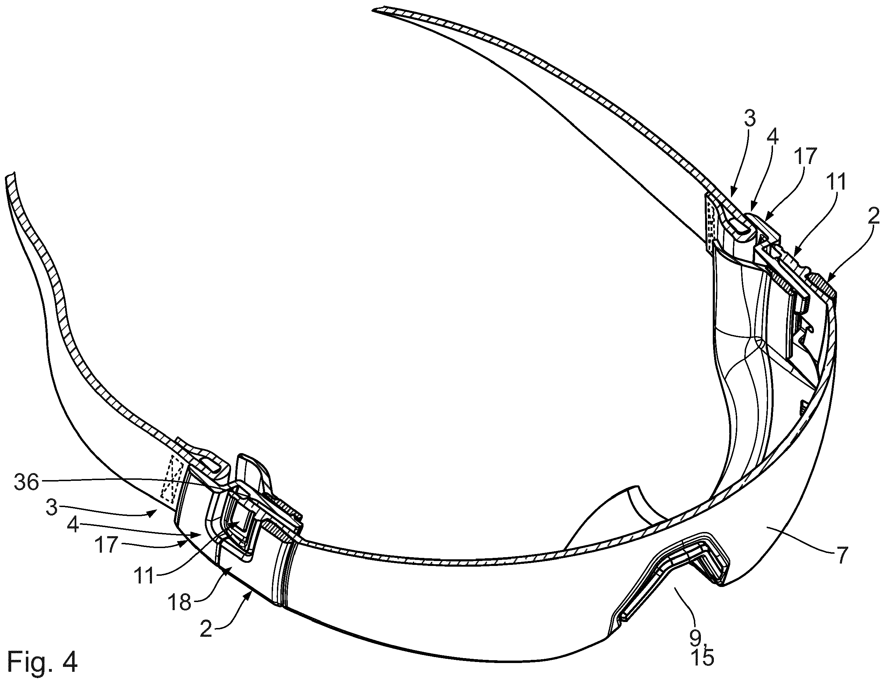

Zunächst Bezug nehmend auf die in den Fig. 1 bis 4 dargestellte, erste Ausführungsform hat eine Brille eine Sichtscheiben-Anordnung 1 und einen Rahmen 2 sowie eine Kopf-Halteanordnung 3. Die Sichtscheiben-Anordnung 1, der Rahmen 2 und die Kopf-Halteanordnung 3 sind zur Bildung der Brille zusammensetzbar und auch wieder voneinander trennbar. Dafür sind zwei Kopplungs-Anordnungen 4 vorgesehen, die seitlich angeordnet sind und konstruktiv identisch sind.

Wenn die Brille bestimmungsgemäß getragen wird, so befindet sich diese vor den Augen des Trägers und bedeckt diese. Die Brille hat so eine Blickrichtung 5. Sie ist bezüglich einer Mittel- bzw. Symmetrieebene 6 symmetrisch ausgeführt, die sich in der Blickrichtung 5 erstreckt und mittig zwischen den Kopplungs-Anordnungen 4 verläuft.

Die Sichtscheiben-Anordnung 1 umfasst eine einzige, durchgängige Sichtscheibe 7 und hat zwei einander gegenüberliegende Seiten-Endbereiche 8. Zwischen den Seiten-Endbereichen 8 hat die Sichtscheiben-Anordnung 1 eine nach unten offene Nasenaussparung 9 zur Aufnahme einer Nase eines Trägers. An die Sichtscheibe 7 schließt sich ein Verbindungssteg 10 an, der um die gesamte Sichtscheibe 7 oder entlang eines Randbereichs derselben läuft und entgegen der Blickrichtung 5 von der Sichtscheibe 7 wegspringt. Im zusammengesetzten Zustand der Brille greift der Verbindungssteg 10 in den Rahmen 2, beispielsweise rastend, ein.

An jeden Seiten-Endbereich 8 der Sichtscheiben-Anordnung 1 schließt sich eine erste Kopplungs-Einrichtung 11 an, die im Wesentlichen laschenartig ausgeführt ist und von der Sichtscheiben-Anordnung 1 entgegen der Blickrichtung 5 wegspringt.

Der Rahmen 2 ist umfangsseitig geschlossen und hat eine Grundform, die prinzipiell der Grundform der Sichtscheiben-Anordnung 1 entspricht. Er hat so zwei einander gegenüberliegende Seiten-Endbereiche 14 und eine mittig zwischen diesen angeordnete Nasenaussparung 15, die nach unten offen ist. Im Wesentlichen ist der Rahmen 2 aus einem formstabilen, in Blickrichtung 5 vorderen Rahmenteil 12 und einem rückseitigen Dichtelement 13 gebildet, das flexibel bzw. nachgiebig ist und im Einsatz an dem Gesicht des Trägers im Wesentlichen dicht anliegt.

Die Kopf-Halteanordnung 3 ist als Kopfband ausgeführt, das zwei einander gegenüberliegende Endbereiche 16 aufweist.

In jedem Endbereich 16 des Kopfbands ist eine zweite Kopplungs-Einrichtung 17 befestigt. Die zweiten Kopplungs-Anordnungen 17 sind dort in endseitigen Schlaufen des Kopfbands angeordnet. Ferner ist in jedem Seiten-Endbereich 14 des Rahmens 2 eine dritte Kopplungs-Einrichtung 18 angeordnet. Die dritten Kopplungs-Einrichtungen 18 sind mit dem Rahmen 2 fest verbunden.

Die Kopplungs-Einrichtungen 11, 17, 18 stehen im zusammengesetzten Zustand der Brille unter Bildung der jeweiligen Kopplungs-Anordnungen 4 miteinander in formschlüssiger Verbindung. Nachdem die Kopplungs-Anordnungen 4 konstruktiv identisch ausgeführt sind, wird nachfolgend hauptsächlich nur noch auf eine Kopplungs-Anordnung 4 eingegangen.

Die ersten Kopplungs-Einrichtungen 11 schließen sich jeweils über eine längliche Anschluss-Stelle 19 an den jeweiligen Seiten-Endbereich 8 der Sichtscheiben-Anordnung 1 an. Gegenüberliegend zu der jeweiligen Anschlussstelle 19 hat jede erste Kopplungs-Einrichtung 11 einen freien hinteren Abstütz-Rand 20. Zwischen der Anschluss-Stelle 19 und dem hinteren Abstütz-Rand 20 erstrecken sich bei jeder ersten Kopplungs-Einrichtung 11 ein oberer Abstütz-Rand 21 und ein unterer Abstütz-Rand 22. Der obere Abstütz-Rand 21 und der untere Abstütz-Rand 22 der jeweiligen ersten Kopplungs-Einrichtung 11 verlaufen im Wesentlichen parallel zueinander und im Wesentlichen senkrecht zu dem zugehörigen hinteren Abstütz-Rand 20 bzw. der Anschluss-Stelle 19.

Benachbart zu der Anschluss-Stelle 19 hat jede erste Kopplungs-Einrichtung 11 einen Halte-Vorsprung 23, der parallel zu der jeweiligen Anschluss-Stelle 19 verläuft. Die Halte-Vorsprünge 23 springen bezogen auf die Symmetrieebene 6 jeweils nach seitlich außen riegelartig vor. Jeder Halte-Vorsprung 23 hat eine Riegel-Fläche 24, die in Blickrichtung 5 bzw. nach vorne gewandt ist.

Die ersten Kopplungs-Einrichtungen 11 sitzen im zusammengesetzten Zustand der Brille jeweils in Teil-Aufnahmen 25, 26 der zweiten Kopplungs-Einrichtung 17 bzw. der dritten Kopplungs-Einrichtung 18 der jeweiligen Kopplungs-Anordnung 4. Die Teil-Aufnahmen 25, 26 einer Kopplungs-Anordnung 4 bilden zusammen eine Gesamt-Aufnahme. Die Gesamt-Aufnahmen bzw. Teil-Aufnahmen 25, 26 sind jeweils bezüglich der Symmetrieebene 6 nach seitlich außen offen. Die Teil-Aufnahmen 25 sind jeweils Bestandteil der dritten Kopplungs-Einrichtungen 18, während die Teil-Aufnahmen 26 jeweils Bestandteil der zweiten Kopplungs-Einrichtungen 17 sind.

Jede Teil-Aufnahme 25 ist durch einen in Blickrichtung 5 vorderen Begrenzungs-Rand 27, einen oberen Begrenzungs-Teilrand 28 und einen unteren Begrenzungs-Teilrand 29 begrenzt. Gegenüber zu dem vorderen Begrenzungs-Rand 29 ist jede Teil-Aufnahme 25 entgegen der Blickrichtung 5 offen. Der obere Begrenzungs-Teilrand 28 und der untere Begrenzungs-Teilrand 29 der jeweiligen Teil-Aufnahme 25 verlaufen im Wesentlichen parallel zueinander und im Wesentlichen senkrecht zu dem vorderen Begrenzungs-Rand 27.

Jede Teil-Aufnahme 26 ist durch einen in Blickrichtung 5 hinteren Begrenzungs-Rand 30, einen oberen Begrenzungs-Teilrand 31 und einen unteren Begrenzungs-Teilrand 32 begrenzt. Gegenüber zu dem hinteren Begrenzungs-Rand 30 ist jede Teil-Aufnahme 26 in Blickrichtung 5 offen. Der obere Begrenzungs-Teilrand 31 und der untere Begrenzungs-Teilrand 32 der jeweiligen Teil-Aufnahme 26 verlaufen im Wesentlichen parallel zueinander und im Wesentlichen senkrecht zu dem hinteren Begrenzungs-Rand 30.

Die oberen Begrenzungs-Teilränder 28, 31 der jeweiligen Kopplungs-Anordnung 4 fluchten im zusammengesetzten Zustand der Brille miteinander und bilden zusammen einen oberen Begrenzungs-Rand. Ebenso fluchten die unteren Begrenzungs-Teilränder 29, 32 der jeweiligen Kopplungs-Anordnung 4 im zusammengesetzten Zustand der Brille miteinander und bilden zusammen einen unteren Begrenzungs-Rand.

Der vordere Begrenzungs-Rand 27 und der hintere Begrenzungs-Rand 30 der jeweiligen Kopplungs-Anordnung 4 verlaufen im Wesentlichen parallel zueinander und sind in Blickrichtung 5 beabstandet zueinander angeordnet.

Die Teil-Aufnahmen 25, 26 der jeweiligen Kopplungs-Anordnung 4 sind jeweils nach innen in Richtung auf die Symmetrieebene 6 durch einen Halte-Körper 33 begrenzt, der laschenartig ausgeführt ist und Bestandteil der zweiten Kopplungs-Einrichtung 17 ist. Jeder Halte-Körper 33 ist gegenüber einer Außenfläche 34 der zweiten Kopplungs-Einrichtung 17 unter Bildung des jeweiligen hinteren Begrenzungs-Rands 30 nach innen versetzt und springt von dort in Blickrichtung 5 vor. Jeder Halte-Körper 33 hat eine plattenartige Halte-Basis 35 und einen von der Halte-Basis 35 vorspringenden Gelenk-Vorsprung 36, der benachbart zu dem jeweiligen hinteren Begrenzungs-Rand 30 angeordnet ist und im Wesentlichen parallel zu diesem verläuft. Jeder Gelenk-Vorsprung 36 springt von der zugehörigen Halte-Basis 35 bezogen auf die Symmetrieebene 6 nach außen vor und verjüngt sich dabei. Die Gelenk-Vorsprünge 36 sind stegartig ausgeführt.

Ferner hat jeder Halte-Körper 33 ein Halte-Mittel 37, das im Wesentlichen parallel zu dem jeweiligen Gelenk-Vorsprung 36 der Kopplungs-Anordnung 4 verläuft und von der jeweiligen Halte-Basis 35 nach innen in Richtung auf die Symmetrieebene 6 unter Bildung einer jeweiligen Halte-Fläche 38 vorspringt. Es ist aus Montagegründen vorteilhaft, wenn jedes Halte-Mittel 37 rampenartig ausgeführt ist und entgegen der Blickrichtung 5 ansteigt. Die Halte-Mittel 37 sind benachbart, aber beabstandet zu einem freien Rand 39 des jeweiligen Halte-Körpers 33 angeordnet. Der Gelenk-Vorsprung 36 und das Halte-Mittel 37 der jeweiligen Kopplungs-Anordnung 4 springen also in einander gegensinnigen Richtungen von der jeweiligen Halte-Basis 35 vor.

Jede dritte Kopplungs-Einrichtung 18 hat ein Außen-Teil 40 und ein benachbart, aber beabstandet zu diesem angeordnetes Innen-Teil 41. Die Außen-Teile 40 und die Innen-Teile 41 sind entsprechend an dem Rahmen 2 angeordnet bzw. ausgebildet. In den Außen-Teilen 40 sind die Teil-Aufnahmen 25 angeordnet.

An jedem Innen-Teil 41 ist ein Halte-Gegenmittel 42 mit einer Halte-Gegenfläche 43 angeordnet, die durch einen von dem Innen-Teil 41 nach außen in Richtung auf das zugehörige Außen-Teil 40 vorspringenden Gegenvorsprung 44 gebildet ist.

Jede dritte Kopplungs-Einrichtung hat eine Durchgangs-Öffnung 48, die durch das jeweilige Außen-Teil 40 und Innen-Teil 41 seitlich begrenzt ist. Die Durchgangs-Öffnungen 48 schließen sich an die jeweilige Teil-Aufnahme 25 an und sind in Blickrichtung 5 offen.

Im zusammengesetzten Zustand der Brille liegen die unteren Abstütz-Ränder 22 der ersten Kopplungs-Einrichtungen 11 an den unteren Begrenzungs-Rändern der Gesamt-Aufnahmen zumindest bereichsweise an, während die oberen Abstütz-Ränder 21 der ersten Kopplungs-Einrichtungen 11 an den oberen Begrenzungs-Rändern der Gesamt-Aufnahmen zumindest bereichsweise anliegen. Ferner liegen die hinteren Abstütz-Ränder 20 an den hinteren Begrenzungs-Rändern 30 zumindest bereichsweise an. Die Halte-Vorsprünge 23 liegen jeweils zumindest bereichsweise an den vorderen Begrenzungs-Rändern 27 an. Insbesondere liegen die Riegel-Flächen 24 an den vorderen Begrenzungs-Rändern 27 zumindest bereichsweise riegelnd an. Die Gelenk-Vorsprünge 36 liegen innenseitig an den ersten Kopplungs-Einrichtungen 11 unter Bildung von Gelenk-Achsen 45 an. Die Halte-Mittel 37 stehen mit den zugehörigen Halte-Gegenmitteln 42 in Verbindung bzw. Kontakt. Insbesondere liegen die Halte-Flächen 38 an den Halte-Gegenflächen 43 der jeweiligen Kopplungs-Anordnung 4 zumindest bereichsweise flächig an.

Vorzugsweise verläuft jede Gelenk-Achse 45 im Wesentlichen in einer Mittelebene 47 der jeweiligen Kopplungs-Anordnung 4 bzw. parallel zu dieser. Vorzugsweise verläuft jede Gelenk-Achse 45 im Wesentlichen parallel zu der Symmetrieebene 6. Vorzugsweise verläuft jede Gelenk-Achse 45 im Wesentlichen senkrecht zu der Blickrichtung 5.

Durch die Aufnahme der ersten Kopplungs-Einrichtungen 11 in den zweiten Kopplungs-Einrichtungen 17 und dritten Kopplungs-Einrichtungen 18 und die an den dritten Kopplungs-Einrichtungen 18 anliegenden Halte-Vorsprünge 23 sind die ersten Kopplungs-Einrichtungen 11 gegenüber den zweiten Kopplungs-Einrichtungen 17 und dritten Kopplungs-Einrichtungen 18 der jeweiligen Kopplungs-Anordnung 4 örtlich festgelegt. Durch die riegelnde Wechselwirkung zwischen den Halte-Mitteln 37 und den Halte-Gegenmitteln 42 der jeweiligen Kopplungs-Anordnung 4 sind die zweiten Kopplungs-Einrichtungen 17 und die dritten Kopplungs-Einrichtungen 18 der jeweiligen Kopplungs-Anordnung 4 zueinander fixiert. Die Halte-Körper 33 befinden sich dabei in ihrer Halte-Stellung.

Nachfolgend wird die Montage der Brille beschrieben. Zunächst werden die zweiten Kopplungs-Einrichtungen 17 in die dritten Kopplungs-Einrichtungen 18 in Blickrichtung 5 eingeschoben. Die Halte-Körper 33 federn dann in ihre Halte-Stellung nach außen, wenn deren Halte-Mittel 37 mit den jeweiligen Halte-Gegenmitteln 42 in Wechselwirkung treten. Anschließend werden die ersten Kopplungs-Einrichtungen 11 entgegen der Blickrichtung 5 durch die dritten Kopplungs-Einrichtungen 18 bzw. die Durchgangs-Öffnungen 48 derart angefügt, dass sich die ersten Kopplungs-Einrichtungen 11 in den Gesamt-Aufnahmen der jeweiligen Kopplungs-Anordnung 4 befinden.

Alternativ werden die ersten Kopplungs-Einrichtungen 11 zunächst durch die dritten Kopplungs-Einrichtungen 18 entgegen der Blickrichtung 5 in die Teil-Aufnahme 25 der jeweiligen Kopplungs-Anordnung 4 eingefügt. Anschließend werden die zweiten Kopplungs-Einrichtungen 17 in rastende Verbindung mit den dritten Kopplungs-Einrichtungen 18 gebracht.

Zur Demontage der Brille werden die Halte-Körper 33 nach seitlich außen durch Aufbringen einer entsprechenden Schwenkkraft bei deren freien Rand 39 in ihre äußere Freigabe-Stellung geschwenkt, so dass die RastVerbindung zwischen dem Halte-Mittel 37 und dem zugehörigen Halte-Gegenmittel 42 aufgelöst wird und eine Trennung der zweiten Kopplungs-Einrichtungen 17 und der dritten Kopplungs-Einrichtungen 18 der jeweiligen Kopplungs-Anordnung 4 möglich ist. Anschließend werden die ersten Kopplungs-Einrichtungen 11 aus den dritten Kopplungs-Einrichtungen 18 geführt.

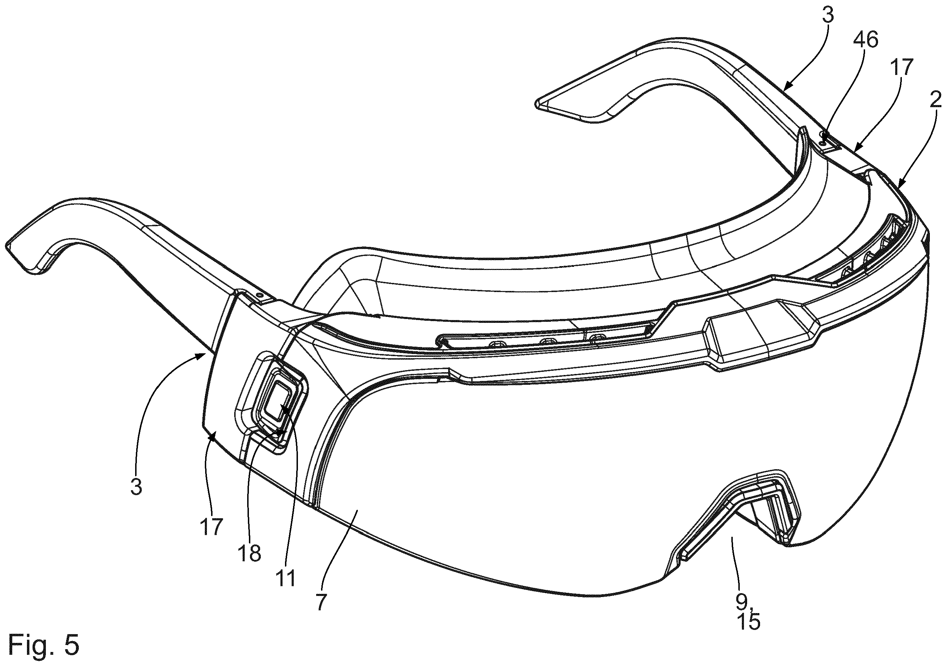

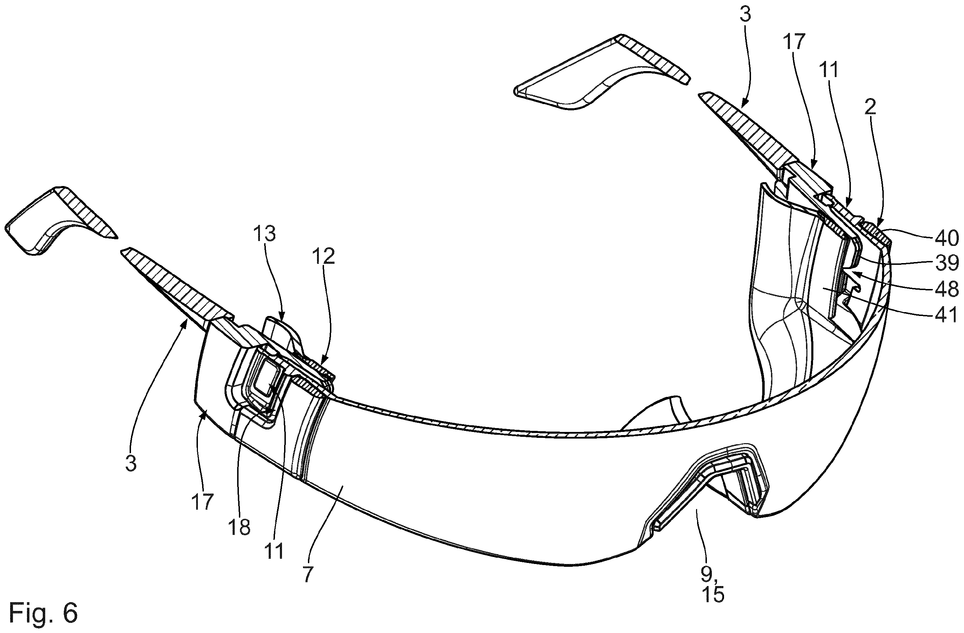

Nachfolgend wird unter Bezugnahme auf die Fig. 5 und 6 die zweite Ausführungsform beschrieben. Im Gegensatz zu der vorherigen Ausführungsform, auf deren Beschreibung verwiesen wird, ist die Kopf-Halteanordnung 3 durch zwei Brillenbügel gebildet, die durch eine Scharnier-Anordnung 46 ein- bzw. ausklappbar sind. Ansonsten bestehen keine Unterschiede.

## Patentansprüche

1. Brille, insbesondere Arbeitsschutzbrille,
a) mit einer Sichtscheiben-Anordnung (1), die
i) zwei einander gegenüberliegende Seiten-Endbereiche (8) aufweist,
b) mit einem Rahmen (2) zum Tragen der Sichtscheiben-Anordnung (1),
c) mit einer Kopf-Halteanordnung (3) zum örtlichen Festlegen der Brille an einem Kopf eines Trägers, und
d) mit zwei Kopplungs-Anordnungen (4) zum koppelnden, lösbaren Verbinden der Sichtscheiben-Anordnung (1), des Rahmens (2) und der Kopf-Halteanordnung (3) miteinander, wobei jede Kopplungs-Anordnung (4) umfasst
i) eine benachbart zu dem jeweiligen Seiten-Endbereich (8) an der Sichtscheiben-Anordnung (1) angeordnete erste Kopplungs-Einrichtung (11),
ii) eine an der Kopf-Halteanordnung (3) angeordnete zweite Kopplungs-Einrichtung (17), die
- eine Kopf-Halteanordnungs-Teil-Aufnahme (26) zur teilweisen Aufnahme der jeweiligen ersten Kopplungs-Einrichtung (11) aufweist, und
- einen zwischen einer Halte-Stellung und einer Freigabe-Stellung verschwenkbaren Halte-Körper (33) mit einem Halte-Mittel (37) aufweist,
- wobei jeder Halte-Körper (33) und jede erste Kopplungs-Einrichtung (11) der jeweiligen Kopplungs-Anordnung (4) im zusammengesetzten Zustand der Brille über mindestens einen Gelenk-Vorsprung (36) miteinander in gelenkiger Verbindung stehen, der eine Gelenk-Achse (45) zum Verschwenken des jeweiligen Halte-Körpers (33) vorgibt, und
iii) eine benachbart zu dem jeweiligen Seiten-Endbereich (8) an dem Rahmen (2) angeordnete dritte Kopplungs-Einrichtung (18), die
- eine Rahmen-Teil-Aufnahme (25) zur teilweisen Aufnahme der jeweiligen ersten Kopplungs-Einrichtung (11) aufweist, und
- ein dem jeweiligen Halte-Mittel (37) zugeordnetes Halte-Gegenmittel (42) zum Zusammenhalten der jeweiligen zweiten Kopplungs-Einrichtung (17) und dritten Kopplungs-Einrichtung (18) aufweist, wenn sich der jeweilige Halte-Körper (33) in seiner Halte-Stellung befindet,
**dadurch gekennzeichnet, dass**
ein zur Demontage zu betätigender Bereich des Halte-Körpers (33) nur von einer Innenseite der Brille aus zugänglich ist.

2. Brille nach Anspruch 1, **dadurch gekennzeichnet, dass** im zusammengesetzten Zustand der Brille die Kopf-Halteanordnungs-Teil-Aufnahme (26) und die Rahmen-Teil-Aufnahme (25) der jeweiligen Kopplungs-Anordnung (4) zusammen eine Gesamt-Aufnahme zum Aufnehmen der jeweiligen ersten Kopplungs-Einrichtung (11) bilden und diese erste Kopplungs-Einrichtung (11) zusammen zumindest bereichsweise, vorzugsweise im Wesentlichen vollständig, umfangsseitig umgeben, wobei vorzugsweise im zusammengesetzten Zustand der Brille jede erste Kopplungs-Einrichtung (11) mit der zugehörigen Gesamt-Aufnahme in formschlüssiger Verbindung steht.

3. Brille nach einem der vorherigen Ansprüche, **dadurch gekennzeichnet, dass** jede erste Kopplungs-Einrichtung (11) einen bezüglich einer Mittelebene (6) der Brille nach seitlich außen vorspringenden Halte-Vorsprung (23) aufweist, der im zusammengesetzten Zustand der Brille blockierend mit der dritten Kopplungs-Einrichtung (18) wechselwirkt und eine Trennung der Sichtscheiben-Anordnung (1) von der jeweiligen dritten Kopplungs-Einrichtung (18) in Blickrichtung (5) der Brille verhindert.

4. Brille nach Anspruch 3, **dadurch gekennzeichnet, dass** der Halte-Vorsprung (23) und das Halte-Mittel (37) der jeweiligen Kopplungs-Anordnung (4) im zusammengesetzten Zustand der Brille in gegensinnigen Richtungen bezüglich einer Mittelebene (47) der jeweiligen Kopplungs-Anordnung (4) vorspringen.

5. Brille nach einem der vorherigen Ansprüche, **dadurch gekennzeichnet, dass** jede erste Kopplungs-Einrichtung (11) im Wesentlichen eckig, vorzugsweise rechteckig, ausgeführt ist.

6. Brille nach einem der vorherigen Ansprüche, **dadurch gekennzeichnet, dass** jede dritte Kopplungs-Einrichtung (18) eine Durchgangs-Öffnung (48) in Blickrichtung (5) der Brille aufweist, durch die die erste Kopplungs-Einrichtung (11) in die Teil-Aufnahme (25, 26) einführbar und ausführbar ist, wobei vorzugsweise jeder Halte-Körper (33) gegenüber einem vorderen Rand der jeweiligen Durchgangs-Öffnung (48) zum manuellen Verschwenken des Halte-Körpers (33) in seine Freigabe-Stellung in Blickrichtung (5) der Brille übersteht.

7. Brille nach einem der vorherigen Ansprüche, **dadurch gekennzeichnet, dass** jeder Halte-Körper (33) als Feder-Halte-Körper ausgeführt ist und sich im freien Zustand in der Halte-Stellung befindet.

8. Brille nach einem der vorherigen Ansprüche, **dadurch gekennzeichnet, dass** der mindestens eine Gelenk-Vorsprung (36) an dem jeweiligen Halte-Körper (33) angeordnet ist.

9. Brille nach einem der vorherigen Ansprüche, **dadurch gekennzeichnet, dass** im zusammengesetzten Zustand der Brille jedes Halte-Mittel (37) innerhalb der jeweiligen dritten Kopplungs-Einrichtung (18) aufgenommen ist.

10. Brille nach einem der vorherigen Ansprüche, **dadurch gekennzeichnet, dass** jedes Halte-Mittel (37) gegenüber einer Halte-Basis (35) des jeweiligen Halte-Körpers (33) unter Bildung einer einem benachbart angeordneten Bereich der Kopf-Halteanordnung (3) zugewandten Halte-Fläche (38) zum Wechselwirken mit der dritten Kopplungs-Einrichtung (18), vorzugsweise nach innen in Richtung auf eine Mittelebene (6) der Brille, vorspringt.

11. Brille nach Anspruch 10, **dadurch gekennzeichnet, dass** jedes Halte-Gegenmittel (42) als Halte-Gegenvorsprung ausgeführt ist, der im zusammengesetzten Zustand der Brille eine der zugehörigen Halte-Fläche (38) zugewandte Halte-Gegenfläche (43) zum Wechselwirken mit dieser Halte-Fläche (38) aufweist.

12. Brille nach einem der vorherigen Ansprüche, **dadurch gekennzeichnet, dass** die zweite Kopplungs-Einrichtung (17) und die erste Kopplungs-Einrichtung (11) im Bereich des jeweiligen Halte-Mittels (37) im zusammengesetzten Zustand der Brille beabstandet zueinander verlaufen, wenn sich der jeweilige Halte-Körper (33) in seiner Halte-Stellung befindet.

13. Brille nach einem der vorherigen Ansprüche, **dadurch gekennzeichnet, dass** im zusammengesetzten Zustand der Brille die Sichtscheiben-Anordnung (1) bei ihren Seiten-Endbereichen (8) innenseitig bereichsweise an der jeweiligen dritten Kopplungs-Einrichtung (18) anliegt.

14. Brille nach einem der vorherigen Ansprüche, **dadurch gekennzeichnet, dass** die Sichtscheiben-Anordnung (1) und die ersten Kopplungs-Einrichtungen (11) einstückig miteinander verbunden sind.

15. Brille nach einem der vorherigen Ansprüche, **dadurch gekennzeichnet, dass** jeder Gelenk-Vorsprung (36) als länglicher Gelenk-Steg ausgeführt ist.

## Claims

1. Goggles, in particular work safety goggles,
a) with a lens arrangement (1), which
i) has two mutually opposing side end regions (8),
b) with a frame (2) to carry the lens arrangement (1),
c) with a head holding arrangement (3) to locally fix the goggles on a head of a wearer, and
d) with two coupling arrangements (4) for a coupling, releasable connection of the lens arrangement (1), the frame (2) and the head holding arrangement (3) to one another, wherein each coupling arrangement (4) comprises
i) a first coupling device (11) arranged adjacent to the respective side end region (8) on the lens arrangement (1),
ii) a second coupling device (17), which is arranged on the head holding arrangement (3) and which
- has a head holding arrangement part receiver (26) to partly receive the respective first coupling device (11), and
- a holding body (33), which is pivotable between a holding position and a release position, with a holding means (37),
- wherein each holding body (33) and each first coupling device (11) of the respective coupling arrangement (4), in the assembled state of the goggles, have an articulated connection to one another by means of at least one joint projection (36), which defines a joint axis (45) for pivoting the respective holding body (33), and
iii) a third coupling device (18) arranged adjacent to the respective side end region (8) on the frame (2), which device
- has a frame part receiver (25) to partially receive the respective first coupling device (11), and
- has a holding counter-means (42), which is associated with the respective holding means (37), to hold together the respective second coupling device (17) and third coupling device (18) when the respective holding body (33) is in its holding position,
**characterized in that**
a region of the holding body (33) to be actuated for disassembly is only accessible from an inside of the goggles.

2. Goggles according to claim 1, **characterized in that,** in the assembled state of the goggles, the head holding arrangement part receiver (26) and the frame part receiver (25) of the respective coupling arrangement (4) together form a total receiver to receive the respective first coupling device (11) and together peripherally surround this first coupling device (11) at least in regions, preferably substantially completely, wherein, in the assembled state of the goggles, each first coupling device (11) preferably has a positive connection to the associated total receiver.

3. Goggles according to any one of the preceding claims, **characterized in that** each first coupling device (11) has a holding projection (23) projecting laterally outwardly with respect to a centre plane (6) of the goggles, the projection, in the assembled state of the goggles, interacting in a blocking manner with the third coupling device (18) and preventing a separation of the lens arrangement (1) from the respective third coupling device (18) in a viewing direction (5) of the goggles.

4. Goggles according to claim 3, **characterized in that** the holding projection (23) and the holding means (37) of the respective coupling arrangement (4), in the assembled state of the goggles, project in opposite directions with respect to a centre plane (47) of the respective coupling arrangement (4).

5. Goggles according to any one of the preceding claims, **characterized in that** each first coupling device (11) is substantially angular, preferably rectangular.

6. Goggles according to any one of the preceding claims, **characterized in that** each third coupling device (18) has a through-opening (48) in a viewing direction (5) of the goggles, through which the first coupling device (11) is introducible into the part receiver (25, 26) and removable therefrom, wherein each holding body (33) preferably projects in the viewing direction (5) of the goggles in relation to a front edge of the respective through-opening (48) for the manual pivoting of the holding body (33) into its release position.

7. Goggles according to any one of the preceding claims, **characterized in that** each holding body (33) is configured as a spring holding body and is in the holding position in the free state.

8. Goggles according to any one of the preceding claims, **characterized in that** the at least one joint projection (36) is arranged on the respective holding body (33).

9. Goggles according to any one of the preceding claims, **characterized in that,** in the assembled state of the goggles, each holding means (37) is received within the respective third coupling device (18).

10. Goggles according to any one of the preceding claims, **characterized in that** each holding means (37) projects, preferably inwardly towards a centre plane (6) of the goggles, in relation to a holding base (35) of the respective holding body (33) with a formation of a holding face (38) facing an adjacently arranged region of the head holding arrangement (3) for interaction with the third coupling device (18).

11. Goggles according to claim 10, **characterized in that** each holding counter-means (42) is configured as a holding counter-projection, which, in the assembled state of the goggles, has a holding counter-face (43) facing the associated holding face (38) for interaction with this holding face (38).

12. Goggles according to any one of the preceding claims, **characterized in that** the second coupling device (17) and the first coupling device (11) run spaced apart from one another in the region of the respective holding means (37) in the assembled state of the goggles when the respective holding body (33) is in its holding position.

13. Goggles according to any one of the preceding claims, **characterized in that,** in the assembled state of the goggles, the lens arrangement (1) at its side end regions (8), in regions on the inside, rests on the respective third coupling device (18).

14. Goggles according to any one of the preceding claims, **characterized in that** the lens arrangement (1) and the first coupling devices (11) are connected to one another in one piece.

15. Goggles according to any one of the preceding claims, **characterized in that** each joint projection (36) is configured as an elongate joint web.

## Revendications

1. Lunettes, en particulier des lunettes de protection de travail, comprenant
a) un ensemble de vitre (1)
i) présentant deux zones d'extrémité latérales (8) opposées,
b) une monture (2) pour supporter l'ensemble de vitre (1),
c) un ensemble support sur tête (3) pour la fixation locale des lunettes sur une tête d'un porteur, et
d) deux ensembles de couplage (4) pour lier l'ensemble de vitre (1), la monture (2) et l'ensemble support sur tête (3) entre eux de manière couplée, détachable, chaque ensemble the couplage (4) comprenant
i) un premier dispositif de couplage (11), avoisinant la zone d'extrémité latérale (8) respective et disposé sur l'ensemble de vitre (1),
ii) un deuxième dispositif de couplage (17), disposé sur l'ensemble support sur tête (3), présentant
- un logement partiel d'ensemble support sur tête (26) pour loger partiellement le premier dispositif de couplage (11) respectif, et
- un corps de retenue (33) avec un moyen de retenue (37), pivotant entre une position de retenue et une position de déblocage,
- chaque corps de retenue (33) et chaque premier dispositif de couplage (11) de l'ensemble de couplage (4) respectif, dans l'état assemblé des lunettes, étant en liaison articulée l'un avec l'autre via au moins une saillie d'articulation (36) qui prédéfinit un axe d'articulation (45) pour pivoter le corps de retenue (33) respectif, et
iii) un troisième dispositif de couplage (18), avoisinant la zone d'extrémité latérale (8) respective et disposé sur la monture (2), présentant
- un logement partiel de monture (25) pour loger partiellement le premier dispositif de couplage (11) respectif, et
- un moyen de retenue équivalent (42) associé au moyen de retenue (37) respectif, pour tenir ensemble le deuxième dispositif de couplage (17) et le troisième dispositif de couplage (18) respectifs, lorsque le corps de retenue (33) respectif se trouve dans sa position de retenue,
**caractérisé en ce qu'**
une zone, à appuyer pour le démontage, sur le corps de retenue (33) n'est accessible que du côté intérieur des lunettes.

2. Lunettes selon la revendication 1, **caractérisé en ce que,** dans l'état assemblé des lunettes, le logement partiel d'ensemble support sur tête (26) et le logement partiel de monture (25) de l'ensemble de couplage (4) respectif, conjointement constituent un logement entier pour loger le premier dispositif de couplage (11) respectif, et conjointement entourent ce premier dispositif de couplage (11), au moins par endroits, préférablement essentiellement entièrement de manière circonférentielle, chaque premier dispositif de couplage (11), dans l'état assemblé des lunettes, préférablement, étant en liaison par engagement positif avec le logement entier associé.

3. Lunettes selon l'une quelconque des revendications précédentes, **caractérisé en ce que** chaque premier dispositif de couplage (11) présente une saillie de retenue (23) saillissant latéralement vers l'extérieur relatif à un plan central (6) des lunettes, interagissant de manière encombrant avec le troisième dispositif de couplage (18), dans l'état assemblé des lunettes, et empêchant une dissociation de l'ensemble de vitre (1) du troisième dispositif de couplage (18) respectif dans la direction du regard (5) des lunettes.

4. Lunettes selon la revendication 3, **caractérisé en ce que** la saillie de retenue (23) et le moyen de retenue (37) de l'ensemble de couplage (4) respectif, dans l'état assemblé des lunettes, saillissent dans des sens inverses relatif à un plan central (47) de l'ensemble de couplage (4) respectif.

5. Lunettes selon l'une quelconque des revendications précédentes, **caractérisé en ce que** chaque premier dispositif de couplage (11) est formé essentiellement de manière anguleuse, préférablement de manière rectangulaire.

6. Lunettes selon l'une quelconque des revendications précédentes, **caractérisé en ce que** chaque troisième dispositif de couplage (18) présente une ouverture de passage (48) dans la direction du regard (5) des lunettes, à travers de laquelle le premier dispositif de couplage (11) peut être introduit dans le logement partiel (25, 26) et enlevé de celui-ci, préférablement chaque corps de retenue (33) saillissant par rapport à un bord antérieur de l'ouverture de passage (48) respective, pour pivoter le corps de retenue (33) manuellement dans sa position de déblocage dans la direction du regard (5) des lunettes.

7. Lunettes selon l'une quelconque des revendications précédentes, **caractérisé en ce que** chaque corps de retenue (33) est réalisé comme un corps de retenue à ressort et se trouve dans l'état libre dans la position de retenue.

8. Lunettes selon l'une quelconque des revendications précédentes, **caractérisé en ce que** l'au moins une saillie d'articulation (36) est disposée sur le corps de retenue (33) respectif.

9. Lunettes selon l'une quelconque des revendications précédentes, **caractérisé en ce que,** dans l'état assemblé des lunettes, chaque moyen de retenue (37) est logé à l'intérieur du troisième dispositif de couplage (18) respectif.

10. Lunettes selon l'une quelconque des revendications précédentes, **caractérisé en ce que** chaque moyen de retenue (37) saillit par rapport à une base de retenue (35) du corps de retenue (33) respectif, formant une surface de retenue (38) orientée face à une zone avoisinante de l'ensemble support sur tête (3), pour interagir avec le troisième dispositif de couplage (18), préférablement vers l'intérieur dans la direction d'un plan central (6) des lunettes.

11. Lunettes selon la revendication 10, **caractérisé en ce que** chaque moyen de retenue équivalent (42) est réalisé comme une saillie de retenue équivalente, présentant une surface de retenue équivalente (43) orientée face à la surface de retenue (38) associée, dans l'état assemblé des lunettes, pour interagir avec ladite surface de retenue (38).

12. Lunettes selon l'une quelconque des revendications précédentes, **caractérisé en ce que** le deuxième dispositif de couplage (17) et le premier dispositif de couplage (11), dans la zone du moyen de retenue (37) respectif, dans l'état assemblé des lunettes, s'étendent de manière espacée l'un de l'autre, lorsque le corps de retenue (33) respectif se trouve dans sa position de retenue.

13. Lunettes selon l'une quelconque des revendications précédentes, **caractérisé en ce que,** dans l'état assemblé des lunettes, l'ensemble de vitre (1), à ses zones d'extrémités latérales (8), sur son côté intérieur, est placé par endroits contre le troisième dispositif de couplage (18) respectif.

14. Lunettes selon l'une quelconque des revendications précédentes, **caractérisé en ce que** l'ensemble de vitre (1) et les premiers dispositifs de couplage (11) sont liés l'un à l'autre en une seule pièce.

15. Lunettes selon l'une quelconque des revendications précédentes, **caractérisé en ce que** chaque saillie d'articulation (36) est réalisé comme une passerelle allongée d'articulation.
